# EUROPÄISCHE PATENTSCHRIFT

(11) **EP 0 533 216 B1**
(45) Veröffentlichungstag und Bekanntmachung des Hinweises auf die Patenterteilung: **22.03.2000**
(21) Anmeldenummer: 92119557.4
(22) Anmeldetag: 29.05.1987
(51) Int. Cl.: C12P 13/06, C12N 15/54, C12N 1/21

(54) **Verfahren zur Herstellung von L-tertiär-Leucin durch Transaminierung**
Process for the preparation of L-tertiairy-leucin by transamination
Procédé de préparation de L-leucine tertiaire par transamination

(30) Priorität: 04.06.1986 DE 3618812
(43) Veröffentlichungstag der Anmeldung: 24.03.1993
(62) Teilanmeldung aus: 87107793.9
(73) Patentinhaber: Hoechst Schering AgrEvo GmbH, 13342 Berlin (DE)
(72) Erfinder: Then, Johann, Dr., W-6230 Frankfurt am Main 80 (DE); Bartsch, Klaus, Dr., W-6374 Steinbach (DE); Deger, Hans-Matthias, Dr., W-6238 Hofheim/Ts. (DE); Grabley, Susanne, Dr., W-6240 Königstein/Ts. (DE); Marquardt, Rüdiger, Dr., W-6000 Frankfurt am Main (DE)

(56) Entgegenhaltungen:
- EP-A- 0 137 372
- EP-A- 0 152 275
- GB-A- 2 161 159

## Beschreibung

Optisch aktive, nicht-proteinogene Aminosäuren haben große Bedeutung aufgrund ihrer bekannten oder potentiellen biologischen Aktivität. Einige werden erfolgreich im pharmazeutischen Bereich eingesetzt, wie L-Dihydroxyphenylalanin (L-Dopa) oder α-Methyldopa, oder finden im Pflanzenschutz Anwendung, wie Phosphinothricin. Andere sind Vorstufen von Pharmazeutika, wie D-Phenylglycin oder β-p-Hydroxyphenylglycin bei der Herstellung der semisynthetischen Penicilline Ampicillin und Amoxycillin. Sie können auch wertvolle Vorstufen für die Synthese von Feinchemikalien sein. In die asymmetrische Synthese von Aminosäurederivaten hat insbesondere auch tertiär-Leucin Eingang gefunden [U. Schöllkopf, Pure and Appl. Chem., 55 1799-1806,(1983)].

Die nicht-proteinogenen optisch aktiven Aminosäuren werden bevorzugt nur auf chemischem Weg hergestellt. Dabei ist nachteilig, daß nicht stereoselektiv gearbeitet werden kann und man das Racemat als Endprodukt erhält. Enzymatische Verfahren haben demgegenüber sehr oft den Vorteil, daß aus einfach herzustellenden Vorstufen mit Hilfe eines Enzymschritts eine chirale Verbindung selektiv synthetisiert werden kann. Dies ist insbesondere vorteilhaft wenn nur eine der beiden stereoisomeren Verbindungen biologisch aktiv ist.

Die Synthese von natürlichen, sogenannten proteinogenen Aminosäuren durch Biotransformation mit Transaminasen ist an sich bekannt. In der Europäischen Patentanmeldung 152 275 wird ein Verfahren zur Herstellung von Phenylalanin durch Transaminierung mit Hilfe eines genetisch modifizierten Mikroorganismus beschrieben, der sich durch Überproduktion der Aminotransferase auszeichnet. Nach der Europäischen Patentanmeldung 135 846 erfolgt die Herstellung von natürlichen L-Aminosäuren derart, daß α-Ketosäuren mit L-Asparaginsäure als Aminogruppendonator in Gegenwart einer Transaminase umgesetzt werden, die aus E. coli isoliert wurde. Es entstehen die der α-Ketosäure entsprechenden L-Aminosäuren sowie Oxalacetat aus der Asparaginsäure.

Die Selektion und Mutation von Mikroorganismen aus der Reihe E. coli, Paracoccus denitrificans, Torula, Rhodotorula und Streptomyces zur Herstellung von L-Phenylalanin aus Phenylbrenztraubensäure mit verbesserter Ausbeute wird in der Deutschen Patentanmeldung DE 3 423 936 dargestellt.

Nicht natürliche vorkommende, sogenannte nicht-proteinogene, Aminosäuren werden bislang nicht durch enzymatische Biotransformation hergestellt.

Es wurde nun gefunden, daß die Synthese der nicht-proteinogenen Aminosäuren L-tertiär-Leucin in sehr guter Ausbeute mit Hilfe der Transaminierung durchgeführt werden kann. Dies ist insofern überraschend, als zwar bekannt war, daß mit Transaminasen verschiedene natürliche proteinogene Aminosäuren synthetisiert werden können, aufgrund der Spezifität der Enzyme jedoch nicht erwartet werden konnte, daß nicht-proteinogene Aminosäuren mit nicht-natürlich vorkommenden α-Ketosäuren als Vorstufe ebenfalls auf diesem Weg hergestellt werden können. Aufgrund dessen ist es überraschend, daß die entsprechenden Vorstufen trotz ihres hydrophoben Restes, die in den Vorstufen zu natürlichen Aminosäuren so nicht vorkommen, von dem aktiven Zentrum der Transaminase toleriert und umgesetzt werden.

Die Erfindung betrifft somit ein Verfahren zur Herstellung von L-tertiär-Leucin das dadurch gekennzeichnet ist, daß 3,3-Dimethyl-2-oxo-butansäure oder jeweils die entsprechenden Salze in Gegenwart von Aminogruppendonatoren transaminiert werden.

Im folgenden wird die Erfindung detailliert erläutert bzw. in den Patentansprüchen definiert.

Enzyme zahlreicher Organismen, beispielsweise aus Mikroorganismen, Pflanzen und tierischen Organen, wie Schweineherz sind in der Lage, α-Ketosäuren durch Transaminierung in natürliche L-Aminosäuren umzuwandeln. Diese Organismen bzw. deren Enzyme können erfindungsgemäß eingesetzt werden. Bevorzugt wird jedoch mit Mikroorganismen gearbeitet, die eine Transaminase besitzen, wie zum Beispiel Mikroorganismen der Gattungen Paracoccus; Alkaligenes, Rhizobium, Pseudomonas, Serratia, Agrobacterium, Streptomyces sowie Enterobakterien. Insbesondere vorteilhaft sind Mikroorganismen, wie beispielsweise Alicaligenes faecalis DSM 4115, Alcaligenes denitrificans DSM 4114, Pseudomonas paucimobilis DSM 4120, Pseudomonas spec. DSM 4119, Serratia plymuthica DSM 4116, Agrobacterium tumefaciens, Escherichia coli DH1, Escherichia coli ATCC 11303, Enterobacter agglomerans DSM 4122, Enterobacter spec. DSM 4121, Paracoccus denitrificans DSM 65, Streptomyces hygroscopicus und Streptomyces viridochromogenes sowie 3 Bodenisolate DSM 4113, DSM 4117 und DSM 4118. Diese Mikroorganismen wurden, sofern sie nicht frei erhältlich oder nacharbeitbar beschrieben waren, bei der Deutschen Sammlung für Mikroorganismen (DSM) hinterlegt.

Man kann höhere Enzymaktivitäten erhalten, wenn man Stämme selektioniert, die gegen Phosphinothricin resistent sind oder Phosphinothricin als alleinige Stickstoffquelle nutzen, beispielsweise Alcaligenes faecalis DSM 4115, Agrobacterium tumefaciens sowie das Bodenisolat DSM 4113. Dies ist vorteilhaft, jedoch nicht unbedingt notwending. Ebenso können durch Selektion und Mutation, in an sich bekannter Weise, gegen steigende Mengen von 3,3-Dimethyl-2-oxobutansäure, Phenylbrenztraubensäure oder deren Salze in den Anzuchtmedien für die weiteren Arbeiten Mikroorganismen ausgewählt werden, die aufgrund ihrer Adaptation an die α-Ketosäure die Transaminierung in besseren Ausbeuten durchführen. 3,3-Dimethyl-2oxo-butansäure bzw. deren Salze sind leicht zugänglich durch Verseifen von Trimethylessigsäure in Gegenwart von Thionylchlorid und KCN nach klassischen Methoden. Die Herstellung von (3-carboxy-3-oxo-propyl)-methylphosphinsäure bzw. deren Salze erfolgt ebenso nach bekannten Methoden (Hans Beyer, Lehrbuch der organischen Chemie, S. Hirzel Verlag, Stuttgart).

Gute Ausbeuten werden auch erreicht, wenn gentechnisch manipulierte Mikroorganismen in dem erfindungsgemäßen Verfahren eingesetzt werden. Besonders bevorzugt wird mit E. coli ATCC 11303 gearbeitet, der mit einem ein tyrB-Gen bzw. ilvE-Gen enthaltenden Plasmid transformiert wurde, wobei das tyrB-Gen für die aromatische Tansaminase und ilvE für die aliphatische Transaminase jeweils in E. coli kodiert. Derartig manipulierte Stämme können beispielsweise nach der Deutschen Patentanmeldung P 36 31 829.9 bzw. P 36 36 722.2 hergestellt werden.

Die Transaminierung kann gleichzeitig mit der Kultivierung erfolgen, wobei dann vorzugsweise mit Mikroorganismen gearbeitet wird, die gegen Phosphinothricin resistent sind, beispielsweise Alcaligenes faecalis DSM 4115 und Agrobacterium tumefaciens. Die Mikroorganismen werden jedoch vorteilhaft in einem für ihr Wachstum optimalen Nährmedium unter entsprechenden günstigen Temperatur- und Belüftungsbedingungen bis zu einem Trockengewicht von etwa 4 bis 60 g/l Nährlösung angezogen. Die für den jeweiligen Mikroorganismus günstigsten Bedingungen sind dem Fachmann entweder bekannt oder können in einfachen Vorversuchen festgestellt werden. Die Zellen werden dann, in der Nährlösung oder von der Nährlösung abgetrennt, zur Aminierung der α-Ketosäuren eingesetzt. Die Transaminierung kann mit ganzen oder auch mit aufgeschlossenen Zellen durchgeführt werden, wobei die üblichen Aufschlußmethoden angewendet werden. Es ist ebenfalls möglich, die Transaminierung mit Zellextrakten, isolierten Gesamtproteinen sowie gereinigten Transaminasen durchzuführen. Aus praktischen Überlegungen beispielsweise aus Kostengründen, wird jedoch bevorzugt mit intakten Zellen gearbeitet. Jedoch kann die Isolierung der Transaminasen aufgrund einer längeren Lebensdauer des Enzyms sowie einer besseren Regulierbarkeit der Reaktion ebenfalls vorteilhaft sein. Es ist weiterhin möglich, die Mikroorganismen oder die Enzyme in fixierter Form einzusetzen. Für die Fixierung kommen die bekannten Verfahren in Betracht, vorteilhaft die Methoden nach den Deutschen Offenlegungsschriften 32 37 341 und 32 43 591.

Die Mikroorganismen bzw. das isolierte Enzymsystem werden in der bevorzugten Ausführungsform in einem physiologischen Puffer suspendiert, so daß deren Transaminaseaktivität nicht nennenswert negativ beeinflußt wird, unter Zugabe der α-Ketosäure und des Aminogruppendonators. Je nach Menge der Mikroorganismen kann die dem Ansatz zugegebene enzymatische Aktivität in Form von Mikroorganismen oder des isolierten Enzymsystems in weiten Bereichen schwanken. Zweckmäßig liegt sie zwischen 10 bis 20.000 µmol/min·l. Vorzugsweise enthält der Ansatz Zellmengen mit einer Enzymaktivität von 1500-2000 µmol/min·l.

Als Aminogruppendonator finden Aminosäuren Anwendung. Welche Aminosäure vorteilhaft eingesetzt wird ist weitgehend von dem Mikroorganismus bzw. dem isolierten Enzymsystem abhängig, was jedoch in kurzen Vorversuchen festgestellt werden kann. Geeignet sind beispielsweise Valin, Leucin, Isoleucin, Methionin, Tyrosin und Phenylalanin, insbesondere Asparagin, Asparaginsäure, Glutamin, Glutaminsäure und Glycin Diese Amimosauren werden in der L-Form, da nur diese erfindungsgemäß verwertet wird, als freie Säure oder geeigneten Salze (entsprechend dem verwendeten Medium) eingesetzt. Zur Herstellung von L-tertiär-Leucin wird 3,3-Dimethyl-2-oxo-butansäure eingesetzt.

Es können auch deren Salze verwandt werden, wobei natürlich Ionen gewählt werden, die die Transaminaseaktivität nicht nennenswert negativ beeinflußen. Bevorzugt sind dies Natrium-, Kalium- und Ammoniumsalze. Der Aminogruppendonator wird in äquimolaren Mengen bzw. im Überschuß zur α-Ketosäure zugegeben. Verhältnisse von 1:1 bis 5:1, vorteilhaft 1:1 bis 2:1, haben sich bewährt.

Die Zugabe der Reaktionskomponenten zum Reaktionsansatz kann als Lösung in Wasser oder durch Zugabe der festen Substanzen gleichzeitig erfolgen. Bevorzugt ist jedoch eine gestaffelte bzw. kontinuierliche Zugabe in Mengen von 0,1-4,5 %, insbesondere 0,2-2 %, jeweils bezogen auf das Gewicht des Reaktionsansatzes, über einen Zeitraum von 1-90 Stunden, vorzugsweise 2-40 Stunden.

Es wird vorteilhaft bei einem pH-Wert zwischen 5 und 9, insbesondere zwischen 7 und 8,5, gearbeitet. Es ist außerdem zweckmäßig, die Transaminierung in einem Temperaturbereich von 10°-65°C, insbesondere 20 bis 50°C, durchzuführen. Bei niedrigeren Temperaturen verläuft die Enzym-Reaktion zunehmend langsamer, während das Enzym bei höheren Temperaturen fortschreitend desaktiviert wird.

Die günstigste Vorgehensweise hängt von dem jeweiligen Mikroorganismus ab und kann in einfachen Vorversuchen leicht festgelegt werden.

Es hat sich besonders bewährt, die Mikroorganismen vor bzw. während der Transaminierungsreaktion zu permeabilisieren. Dies kann durch Zugabe geeigneter Agenzien, wie Toluol, Cetyltrimethylammoniumbromid, Dimethylsulfoxid etc., zum Inkubationsmedium erfolgen.

Die anschließenden Beispiele dienen dazu, die vorgestellte Erfindung weiter zu illustrieren. Prozentangaben beziehen sich, wenn nicht anders angegeben, auf das Gewicht.

### Beispiel 1

### Anzucht und Aufarbeitung der Mikroorganismen

Die genannten hinterlegten und frei erhältlichen Bakterien wurden in 400 ml Flüssigkulturen in LB-Medium [Luria-Bertani-Medium: 10 g Bacto- Trypton/5 g Bacto- Yeast-Extrakt/10 g NaCl pro Liter, (pH 7,5)] oder M9- Minimalmedium [6 g Na₂HPO₄/3 g KH₂PO₄/0,5 g NaCl/1 g NH₄Cl/2 ml 1 M MgSO₄ + 10 ml 20 % Glucose + 0,1 ml 1 M CaCl₂ + 1 ml 1% Vitamin B1 (Thiamin) pro Liter (pH 7,4)] bei 30°C (alle Bakterien außer E. coli) bzw. 37°C (E. coli, DH1) über Nacht kultiviert.
Anschließend wurden die Bakterien abzentrifugiert und die Zellpellets mehrfach in 10 mM Na₂HPO₄, 10 mM NaCl (pH = 7,0) (Waschpuffer) gewaschen und schließlich in 5 ml Waschpuffer pro 3 g Zellpellet suspendiert. Die Zellen wurden durch 5 x 1 min. Ultrabeschallung aufgeschlossen und die Zelltrümmer danach abzentrifugiert. Die so gewonnenen Lysat-Überstände können mehrere Monate bei -20°C aufbewahrt werden.

### Beispiel 2

### Proteinisolierung

Zur Proteinisolierung wurden je 5 ml der Lysat-Überstände mit Waschpuffer auf 50 ml aufgefüllt und die Proteine durch Zugabe von Ammoniumsulfat auf 65 % ausgefällt. Nach dem Abzentrifugieren über 15 min. bei 10000 g wurden die Proteinpellets in je 5 ml 10 mM Na₂HPO₄ (pH 7,0) 1 mM EDTA, 2 % Glycerin, 1 mM Dithiothreitol (DTT) resuspendiert. Diese Suspension kann ebenfalls bei -20°C aufbewahrt werden. Um einen besseren Reinigungseffekt zu erzielen, wurden die Proteine teilweise zweimal mit Ammoniumsulfat gefällt.

Proteinbestimmungen wurden nach der Biuret-Methode vorgenommen. Der Proteingehalt in den nach obigem Protokoll durchgeführten Präparationen lag meist zwischen 5 und 10 mg/ml. Zur partiellen Reinigung der Transaminasen aus Alcaligenes faecalis DSM 4115 und aus DSM 4113 wurden die Proteine mit 25 % bis 75 % Ammoniumsulfat, jeweils in 10 %-Schritten zugegeben, fraktioniert gefällt und die Einzelfraktionen auf Transaminase-Aktivität getestet (siehe unten). Die Proteinfraktion mit der größten spezifischen Aktivität wurden auf eine Sephadex G 100-Gelfiltrationssäule aufgetragen und mit 10 mM Na₂HPO₄ (pH 7,0) eluiert. Die Eluat-Fraktionen mit der höchsten spezifischen Transaminase-Aktivität wurde durch erneute Ammoniumsulfat-Fällung konzentriert und auf 5 mg/ml in 10 mM Na₂HPO₄ (pH 7,0), 1 mM EDTA, 2 % Glycerin, 1 mM DTT aufgenommen. Die Molekulargewichte der (®Sephadex G 100) Polydextran-Säulenfraktionen wurden durch Vergleich mit Molekulargewicht-Standard-Proteinen bestimmt. Die Reinheit der isolierten Transaminase-Fraktionen konnte durch Elektrophorese von Proteinproben in 10 %igen SDS/Polyacrylamid-Gelen überprüft werden.

### Beispiel 6

### Selektion von Escherichia coli ATCC 11303

Escherichia coli ATCC 11303 wurde nach konventionellen Methoden kultiviert und mit N-Methyl-N-nitro-N-nitroguanidin (MNG) mutagenisiert gemäß E. Adelberg et. al., Biochem. Biophys. Res. Comm. 18, 788 (1965). Die mit MNG behandelten Zellen wurden auf einen autoklavierten Agar mit folgender Zusammensetzung ausgestrichen:

| | |
|---|---|
| Fumarsäure | 5 g/l |
| Fleischextrakt | 20 g/l |
| Asparaginsäure | 20 g/l |
| KH₂PO₄ | 2 g/l |
| MgSO₄ · 7 H₂O | 0,5 g/l |
| CaCl₂ · 2 H₂O | 0,1 g/l |
| Agar | 20 g/l |

pH 7,2 wurde mit Natronlauge eingestellt.

Eine sterilfiltrierte Lösung von Phenylpyruvat wurde in den noch heißen Agar eingegossen, so daß eine Endkonzentration von 24 g/l Phenylpyruvat erreicht wurde. Die Platten wurden bei 37 °C 4 Tage inkubiert. Kolonien mit einem Durchmesser von >1 mm wurden isoliert. 20 % der wachsenden Stämme hatten eine erhöhte Transaminaseaktivität im Vergleich zum Ausgangsstamm.

Die Transaminaseaktivitätsbestimmung wurde mit dem Sigma Test-kit G 0390 durchgeführt.

### Beispiel 7

### a. Isolierung und Verdauung des Cosmids pIMS 6026 aus E. coli

Das Cosmid pIMS6026 leitet sich von dem Cosmid pLAFR1 (ATCC 37167) dadurch ab, daß in dessen einzige EcoRI Schnittstelle das handelsübliche EcoRI Fragment kloniert wurde, auf dem das Kanamycin-Resistenzgen des Transposons Tn 903 liegt (Pharmacia, Upsala, Schweden). Durch Verdauung mit BamHI und anschließende Religation kann der größte Teil des handelsüblichen EcoRI Fragments deletiert werden, so daß nur ein kurzes Stück DNA als Insertion zurückbleibt, in der eine BamHI-Schnittstelle von 2 EcoRI-Schnittstellen flankiert wird.

Zur Isolierung des Cosmids pIMS 6026 aus E. coli HB101 wurde entweder nach Humphreys et al. vorgegangen [Biochem. Biophys. Acta 383, 457-63 (1975)] oder eine alkalische Lyse nach Birnboim und Doly [Nucleic Acids Res. 7, 1513 (1979)] im 10-fach größeren Maßstab durchgeführt. In jedem Fall wurde die Plasmid-DNA mindestens einmal durch CsCl/EtBr-Dichtegradientenzentrifugation gereinigt.

Das Cosmid pIMS 6026 wurde vollständig mit dem Restriktionsenzym BamHI verdaut, wobei nach den Angaben des Herstellers, New England Biolabs, verfahren wurde. Zur Überprüfung der Vollständigkeit dieser Verdauung wurde ein Aliquot des Restriktionsansatzes auf ein 0,8 %iges Agarosegel aufgetragen und der Elektrophorese unterworfen. Das Erscheinen nur einer Bande nach Anfärben mit Ethidiumbromid und Bestrahlung mit kurzwelligem UV-Licht (254nm) diente als Hinweis auf eine vollständige Verdauung. Von der verdauten Cosmid-DNA wurde das Restriktionsenzym durch Phenolisierung entfernt, die DNA mittels Ethanol gefällt, mit 70 %igem Ethanol gewaschen und nach Trocknen unter Vakuum in einem geeigneten Volumen an TE-Puffer(10 mM Tris; 1 mM EDTA, pH 8,0) aufgenommen. Wahlweise wurde noch eine Behandlung mit alkalischer Phosphatase nach den Angaben des Herstellers, Boehringer Mannheim, durchgeführt. Nach Zugabe von 1 µl an alkalischer Phosphatase (CIP) wurde 30 Minuten lang bei 37°C inkubiert, das Enzym durch Phenolisierung aus dem Reaktionsansatz entfernt und die DNA, wie oben beschrieben, gereinigt. Schließlich wurde sie in TE-Puffer resuspendiert.

### b. Partielle Verdauung der DNA aus E. coli ATCC 11303

Die Isolierung der Gesamt-DNA aus E. coli ATCC 11303 wurde nach der Methode von Marmur in J. Mol. Biol. 53, 155-162, (1961) durchgeführt. Die isolierte Gesamt-DNA wurde mit dem Restriktionsenzym Sau3A partiell verdaut, so daß hauptsächlich Fragmente in einem Größenbereich von 20-30kb entstanden. Dazu wurde in Vorversuchen das hierfür optimale Verhältnis von DNA und Enzym sowie die optimale Einwirkdauer des Enzyms auf die DNA festgestellt. Die entsprechende Vorgehensweise ist in dem von der Firma BRL herausgegebenen Heft "focus", Vol. 7, Nr 2 (1985) auf Seite 3 beschrieben. Nach Ablauf der als optimal bestimmten Reaktionszeit wurde das Enzym durch Erhitzen auf 65°C über einen Zeitraum von 10 Minuten zerstört und die Bildung von DNA-Fragmenten im gewünschten Größenbereich durch Agarose-Gelelektrophorese mit geeigneten DNA-Markern, z.B. mit EcoRI-verdauter DNA des Phagen λ, überprüft.

### c. Ligation der Restriktionsansätze

Mit Sau3A partiell verdaute Gesamt-DNA aus E. coli ATCC 11303 wurde mit pIMS 6026 Cosmid-DNA, die mit BamHI vollständig gespalten und mit alkalischer Phosphatase behandelt worden war, in einem molaren Verhältnis von etwa 1 : 5 zusammengegeben. Die resultierende Mischung wurde mit einem mehrfach konzentrierten Puffer nach den Angaben von New England Biolabs so versetzt, daß eine für das Enzym T4-DNA-Ligase optimale Ionenkonzentration resultierte, und mit 1 µl des Enzyms mindestens 14 Stunden lang bei 16°C inkubiert. Das gesamtvolumen des Ansatzes betrug dabei 50 µl mit einer Gesamt-DNA-Konzentration von 20 µg/ml.

### d. Verpackung in λ-Phagen

Nach erfolgter Ligase-Reaktion wurde nach Beispiel 3 erhaltene DNA in vitro in die Köpfe von λ-Phagen verpackt. Die dafür notwendigen Extrakte aus zwei verschiedenen Bakterienstämmen können nach Hohn, B., in Wu, R., editor: Recombinant DNA, Methods in Enzymology, Vol. 68, Academic Press, New York, S.299-309 (1979) gewonnen werden oder von Boehringer Mannheim oder Amershan Buchler, Braunschweig, bezogen werden.
3µl der nach Beispiel 3 erhaltenen Mischung wurden mit unmittelbar zuvor aufgetauten Bakterien-Extrakten der Firma Amersham unter Eiskühlung gründlich vermischt. Die Mischung wurde 30-60 Minuten bei 20°C inkubiert und anschließend 200µl SM-Puffer (100 mM NaCl, 10 mM MgSO₄, 50 mM Tris-HCl (pH 7,5), 0,01 % Gelatine) zugegeben. Diese Mischung wurde entweder direkt in eine Transduktions-Reaktion eingesetzt oder nach Zugabe von 10 µl Chloroform bis zur späteren Verwendung bei 4°C aufbewahrt.

### e. Transduktion von E. coli DG 30

5 ml L-Broth, bestehend aus 1 % Bacto-Trypton, 0,5 % Hefeextrakt und 0,5 % NaCl, wurden 0,4 % Maltose zugesetzt und mit 50 µl einer Flüssigkultur von E. coli DG 30 in der stationären Wachstumsphase beimpft. Es wurde 12 Stunden bei 37°C inkubiert, bis die frühe stationäre Phase erreicht war. Die Bakterien wurden abzentrifugiert und vorsichtig in 2,5 ml einer wäßrigen Lösung, die 10 mmolar an MgCl₂ war, resuspendiert. 10 µl der Mischung gemäß Beispiel 4 wurden mit 20 µl der konzentrierten Bakteriensuspension versetzt und 50 Minuten bei Raumtemperatur inkubiert.

Anschließend wurde 200 µl L-Broth hinzugegeben und 1 Stunde bei 37°C inkubiert, wobei die Mischung gelegentlich geschüttelt wurde.
Jeweils 50 µl des Ansatzes wurden auf L-Broth-Agar, der 20 µg/ml Tetracyclin enthielt, ausplattiert. Die Platten wurden mindestens 12 Stunden bei 37°C bebrütet. Bei der beschriebenen Vorgehensweise konnten aus einem Ansatz durchschnittlich 1000 Kolonien erhalten werden.

### f. Selektionierung von E. coli DG 30 mit einem aspC- oder ilvE- oder tyrB-Gen.

Rund 800 Kolonien, die nach Transduktion von E. coli DG 30 nach dem beschriebenen Verfahren auf L-Broth-Agar, der 20 µg/ml Tetracyclin enthielt, erhalten worden waren, wurden auf Minimalagar "gepickt". Der Minimalagar bestand aus M9-Medium mit Glukose (Miller, Experiments in Molecular Genetics, Cold Spring Harbor, 1972), das durch die Aminosäuren Isoleucin, Leucin, Valin, Asparaginsäure und Phenylalanin supplementiert worden war. Die Aminosäure Tyrosin, die der Stamm DG 30 gleichfalls nicht mehr synthetisieren kann, wurde dem Medium jedoch nicht zugegeben. Von den 800 "gepickten" Kolonien konnten 7 auf dem Minimalmedium anwachsen.

Zur Unterscheidung der drei möglichen Gene aspC, ilvE und tyrB in E. coli DG 30 wurden diese 7 Kolonien wiederum auf oben genanntes Minimalmedium "gepickt", das mit den aufgeführten Aminosäuren supplementiert war, bis auf jeweils eine, für die eine der Transaminasen, die von einem der Gene codiert wird, Substratspezifität zeigt.
Das Ergebnis ist in der nachstehenden Tabelle aufgeführt:

| Klon | Minimalmedium, supplementiert bis auf vermutliches | | | | |
|---|---|---|---|---|---|
| | Asp | Leu | Ile | tyr | Gen |
| 1 | + | + | - | + | tyrB |
| 2 | + | + | - | + | tyrB |
| 3 | - | +- | + | +- | ilvE |
| 4 | - | +- | + | +- | ilvE |
| 5 | + | + | - | + | tyrB |
| 6 | + | + | - | + | tyrB |
| 7 | - | +- | + | +- | ilvE |
| + = gutes Wachstum +- = schlechtes Wachstum - = kein Wachstum | | | | | |

### g. Lokalisierung des tyrB-Gens

Durch Minilyse nach Maniatis et al., Cold Spring Harbor, Seiten 366-370 (1982), wurde Cosmid-DNA der Klone 1 bis 7, die gemäß Beispiel 6 erhalten wurden, gewonnen. Anschließend wurde diese Cosmid-DNA in E. coli DH1 (ATCC 33849) eingeschleust, woraus sie in guten Ausbeuten wieder reisoliert werden konnte.
Es wurde Plasmid-DNA, die ursprünglich aus dem Klon 3 von E. coli DG 30 (siehe Beispiel 6) gewonnen wurde, aus dem mit dieser DNA transformierten Stamm E. coli DH 1 isoliert und mit den Restriktionsenzymen SalI und SmaI, den Angaben des Herstellers, New England Biolabs, folgend, vollständig verdaut. Gleichfalls vollständig mit ClaI verdaut wurde der Vektor pAT 153, der anschließend noch einer Behandlung mit alkalischer Phosphatase unterzogen wurde. Die beiden DNAs wurden zusammengegeben, nach der in Beispiel 4 bereits beschriebenen Art und Weise miteinander ligiert und kompetente Zellen des Stammes E. coli ATCC 11303 mit einem Aliquot des Ligase-Ansatzes, z.B. 10 µl, transformiert. Resistente Kolonien wurden auf L-Broth-Platten, die 50 µg/ml Ampicillin enthielten, selektioniert und durch "replica plating" auf L-Broth-Platten mit 20 µg/ml Tetracyclin auf Markerinaktivierung und damit Einbau getestet. Aus Kolonien, die den Phäntotyp AprTcs aufwiesen,w urde durch Minilyse Plasmid-DNA isoliert und durch vollständige Verdauung mit dem Restriktionsenzym ClaI das Vorhandensein von ClaI-Fragmenten in dem Vektor pAT153 überprüft.

### h. Überprüfung der Transaminase-Aktivität

Die gemäß Beispiel 7 erhaltenen Klone wurden mittels APPAT-Test (Aspartat-Phenylpyruvat-Aminotransferase Assay, Sigma Testkit GO390, wobei α-Ketoglutarat durch Phenylpyruvat ersetzt wurde) auf die Aktivität der aromatischen Transaminase, also des Genprodukts von tyrB, getestet. Als Vergleich diente der nicht transformierte Ausgansstamm E. coli ATCC 11303. Dabei konnte in einem Fall eine deutliche Zunahme an tyrB-Aktivität, und zwar um den Faktor 5 bis 10, gegenüber dem Ausgangsstamm E. coli ATCC 11303, gemessen werden.

Durch Agarose-Gelelektrophorese unter Verwendung geeigneter Marker konnte gezeigt werden, daß in dem Stamm, der erhöhte tyrB-Genaktivität aufwies, ein pAT 153-Vektor enthalten war, der ein rund 2,7 MD großes ClaI-Fragment eingebaut enthielt. Wurde mit der isolierten Plasmid-DNA erneut der plasmidlose Stamm E. coli ATCC 11303 tranformiert, so konnte in jedem Fall eine Zunahme der tyrB-Genaktivität um den Faktor 5-10 beobachtet werden.

Die Transformation von E. coli ATCC 11303 mit dem ilvE-Gen erfolgt in analoger Weise.

### Beispiel 8

### Herstellung von L-tertiär-Leucin

a. Ein gemäß Beispiel 6 selektionierter Stamm von Escherichia coli ATCC 11303 wurde in folgender Nährlösung kultiviert:

| | |
|---|---|
| Fumarsäure | 10 g/l |
| Fleischextrakt | 20 g/l |
| Asparaginsäure | 8 g/l |
| KH₂PO₄ | 2 g/l |
| MgSO₄ · 7 H₂O | 0,5 g/l |
| CaCl₂ · 2 H₂O | 0,1 g/l |
| 3,3-Dimethyl-2-oxo-butansäure | 4 g/l |

pH 7,4 wurde mit Natronlauge eingestellt.
Nach 48 h Wachstum bei 37 °C wurden die Zellen abzentrifugiert. Im Überstand wurden mit Hilfe der HPLC auf einer RPC-8 Säule (mobile Phase: Gradient aus 100 mM Na-Acetat (pH 7,2) und Methanol) 0,9 g/l L-2-Amino-3,3-dimethyl-butansäure (tertiär-Leucin) bestimmt.
b. Zellmaterial wurde wie in Beispiel 8a angezogen und in einer Lösung aus 10 g/l Asparaginsäure, 4 g/l 3,3-Dimethyl-2-oxo-butansäure in 10 mmol/l Tris-HCl-Puffer (pH 7,4) unter Schütteln inkubiert. Nach 24 h bei 37°C konnten mit Hilfe der HPLC 1,9 g/l L-2-Amino-3,3-dimethyl-butansäure gemessen werden.

## Patentansprüche

1. Verfahren zur Herstellung von L-tertiär-Leucin, dadurch gekennzeichnet, daß 3,3-Dimethyl-2-oxo-butansäure bzw. die Salze dieser Verbindungen, jeweils in Gegenwart von Aminosäuren als Aminogruppendonatoren transaminiert werden.

2. Verfahren nach Anspruch 1, dadurch gekennzeichnet, daß mit Hilfe von Mikroorganismen transaminiert wird.

3. Verfahren nach Anspruch 2, dadurch gekennzeichnet, daß mit Hilfe von Mikroorganismen der Gattungen Paracoccus, Alkaligenes, Pseudomonas, Serratia, Agrobacterium und Streptomyces sowie Enterobakterien transaminiert wird.

4. Verfahren nach Anspruch 1 und Anspruch 2, dadurch gekennzeichnet, daß mit Hilfe von Alcaligenes faecalis DSM 4115, Alcaligenes denitrificans DSM 4114, Pseudomonas paucimobilis DSM 4120, Pseudomonas spec. DSM 4119, Serratia plymuthica DSM 4116, Agrobakterium tumefaciens, Escherichia coli DH1, Escherichia coli ATCC 11303, Enterobacter agglomerans DSM 4122, Enterobacter spec. DSM 4121, Paracoccus denitrificans DSM 65, Streptomyces hygroscopicus und Streptomyces viridochromogenes sowie 3 Bodenisolaten DSM 4113, DSM 4117 und DSM 4118 transaminiert wird.

5. Verfahren nach Anspruch 2, dadurch gekennzeichnet, daß mit Hilfe von gentechnisch manipulierten Mikroorganismen transaminiert wird.

6. Verfahren nach Anspruch 5, dadurch gekennzeichnet, daß mit Hilfe von E. coli ATCC 11303, der mit einem ein tyrB-Gen oder ein ilvE-Gen enthaltenden Plasmid transformiert ist, transaminiert wird.

7. Verfahren nach einem oder mehreren der Ansprüche 1 bis 6, dadurch gekennzeichnet, daß mit Zellextrakten, isolierten Gesamtproteinen oder mit gereinigten Transaminasen transaminiert wird.

8. Verfahren nach einem oder mehreren der Ansprüche 1 bis 7, dadurch gekennzeichnet, daß Aminogruppendonator und α-Ketosäure im Verhältnis 1:1 bis 5:1 eingesetzt werden.

9. Verfahren nach Anspruch 8, dadurch gekennzeichnet, daß Aminogruppendonator und α-Ketosäure im Verhältnis 1:1 bis 2:1 eingesetzt werden.

10. Verfahren nach einem oder mehreren der Ansprüche 1 bis 9, dadurch gekennzeichnet, daß die Transaminierung bei einem pH-Wert im Bereich von 5 bis 9 durchgeführt wird.

11. Verfahren nach Anspruch 10, dadurch gekennzeichnet, daß die Transaminierung bei einem pH-Wert im Bereich von 7 bis 8,5 durchgeführt wird.

## Claims

1. A process for preparing L-tertiary leucine, which comprises transaminating 3,3-dimethyl-2-oxobutanoic acid or salt of this compound, in each case in the presence of amino acids as amino group donors.

2. The process as claimed in claim 1, wherein transamination is effected by means of microorganisms.

3. The process as claimed in claim 2, wherein transamination is effected by means of microorganisms from the genera Paracoccus, Alcaligenes, Pseudomonas, Serratia, Agrobacterium and Streptomyces, as well as enterobacteria.

4. The process as claimed in claims 1 and 2, wherein transamination is effected by means of Alcaligenes faecalis DSM 4115, Alcaligenes denitrificans DSM 4114, Pseudomonas paucimobilis DSM 4120, Pseudomonas spec. DSM 4119, Serratia plymuthica DSM 4116, Agrobacterium tumefaciens, Escherichia coli DH1, Escherichia coli ATCC 11303, Enterobacter agglomerans DSM 4122, Enterobacter spec. DSM 4121, Paracoccus denitrificans DSM 65, Streptomyces hygroscopicus and Streptomyces viridochromogenes as well as 3 soil isolates DSM 4113, DSM 4117 and DSM 4118.

5. The process as claimed in claim 2, wherein transamination is effected by means of genetically manipulated microorganisms.

6. The process as claimed in claim 5, wherein transamination is effected by means of E. coli ATCC 11303 which has been transformed with a plasmid containing a tyrB gene or an ilvE gene.

7. The process as claimed in one or more of claims 1 to 6, wherein transamination is effected using cell extracts, isolated total proteins or purified transaminases.

8. The process as claimed in one or more of claims 1 to 7, wherein amino group donor and α-keto acid are employed in a ratio of 1:1 to 5:1.

9. The process as claimed in claim 8, wherein amino group donor and α-keto acid are employed in a ratio of 1:1 to 2:1.

10. The process as claimed in one or more of claims 1 to 9, wherein the transamination is carried out at a pH in the range from 5 to 9.

11. The process as claimed in claim 10, wherein the transamination is carried out at a pH in the range from 7 to 8.5.

## Revendications

1. Procédé de préparation de L-leucine tertiaire, caractérisé en ce que l'on effectue une transamination de l'acide 3,3-diméthyl-2-oxobutanoïque ou des sels de ce composé, en présence chaque fois d'acides aminés comme donneurs de groupes amino.

2. Procédé selon la revendication 1, caractérisé en ce que la transamination se fait à l'aide de micro-organismes.

3. Procédé selon la revendication 2, caractérisé en ce que la transamination se fait à l'aide de micro-organismes des genres Paracoccus, Alcaligenes, Pseudomonas, Serratia, Agrobacterium et Streptomyces, ainsi que des entérobactéries.

4. Procédé selon la revendication 1 et la revendication 2, caractérisé en ce que la transamination se fait à l'aide de Alcaligenes faecalis DSM 4115, Alcaligenes denitrificans DSM 4114, Pseudomonas paucimobilis DSM 4120, Pseudomonas sp. DSM 4119, Serratia plymuthica DSM 4116, Agrobacterium tumefaciens, Escherichia coli DH1, Escherichia coli ATCC 11303, Enterobacter agglomerans DSM 4122, Enterobacter sp. DSM 4121, Paracoccus denitrificans DSM 65, Streptomyces hygroscopicus et Streptomyces viridochromogenes, ainsi que 3 isolats de sol DSM 4113, DSM 4117 et DSM 4118.

5. Procédé selon la revendication 2, caractérisé en ce que la transamination se fait à l'aide de micro-organismes manipulés par génie génétique.

6. Procédé selon la revendication 5, caractérisé en ce que la transamination se fait à l'aide de E. coli ATCC 11303 qui est transformé avec un plasmide contenant un gène tyrB ou un gène ilvE.

7. Procédé selon l'une ou plusieurs des revendications 1 à 6, caractérisé en ce que la transamination se fait avec des extraits cellulaires, des protéines totales isolées, ou avec des transaminases purifiées.

8. Procédé selon l'une ou plusieurs des revendications 1 à 7, caractérisé en ce que le donneur de groupes amino et l'a-cétoacide sont utilisés dans un rapport de 1:1 à 5:1.

9. Procédé selon la revendication 8, caractérisé en ce que le donneur de groupes amino et l'a-cétoacide sont utilisés dans un rapport de 1:1 à 2:1.

10. Procédé selon l'une ou plusieurs des revendications 1 à 9, caractérisé en ce que la transamination se fait à un pH dans la plage de 5 à 9.

11. Procédé selon la revendication 10, caractérisé en ce que la transamination se fait à un pH dans la plage de 7 à 8,5.
